# EUROPEAN PATENT SPECIFICATION

(11) **EP 0 470 251 B1**
(45) Date of publication and mention of the grant of the patent: **07.02.1996**
(21) Application number: 90906353.9
(22) Date of filing: 23.04.1990
(51) Int. Cl.: A61K 31/557, A61K 9/08, A61K 9/107, A61K 9/127, A61K 47/34

(54) **LIQUID PROSTAGLANDIN COMPOSITION**
FLÜSSIGE PROSTAGLANDINZUSAMMENSETZUNG
COMPOSITION LIQUIDE DE PROSTAGLANDINE

(30) Priority: 25.04.1989 JP 103449/89
(43) Date of publication of application: 12.02.1992
(73) Proprietor: THE GREEN CROSS CORPORATION, Osaka-shi Osaka (JP)
(72) Inventor: IMAGAWA, Takashi; The Green Cross Corporation, 2-1180-1 Shodaiohtani Hirakata-shi Osaka (JP)
(74) Representative: von Kreisler, Alek, Dipl.-Chem.
(86) International application number: JP9000525
(87) International publication number: WO9012576

(56) References cited:
- JP-A- 6 013 779
- JP-A-59 206 349
- JP-A-59 216 820
- JP-A-61 502 817
- STN FILE SERVER, & FILE CA, & CHEMICAL ABSTRACTS, vol. 106, no. 6, abstract no. 38360n, Columbus, Ohio, US; T.K. LAW et al.: "Some chemically modified poloxamer hydrogels: controlled release of prostaglandin-E2 and testosterone"
- DERWENT ABSTRACT, (WPI Acc No:80-18422C/11), CA-A-1072413

## Description

The present invention relates to stable liquid compositions containing prostaglandin.

### [Background Art]

It has been generally known that prostaglandins possess pharmacological activities. For example, they stimulate smooth muscle, have hypotensive action and anti-lipolysis action, and inhibit platelet aggregation. For this reason, prostaglandins are useful for the treatment of hypertension, thrombosis, asthma and gastro-intestinal disorders such as ulcer, labor pain induction and abortion for pregnant mammals, and prevention of arteriosclerosis. They are liposoluble substances which can be obtained in a very small amount from various organs of animals capable of prostaglandin secretion in bodies.

Prostaglandins respectively possess biological actions such as smooth muscle (e.g. myometrial muscle, muscle of resected small intestine, etc.)-contracting action, hypotensive action, hypertensive action, anti-lipolyis action, gastric juice secretion-inhibitory action, action on central nerves system, platelet adhesiveness reducing action, platelet aggregation and thrombosis inhibiting action, epidermal proliferation action, keratinization stimulating action, and so on.

However, the chemical unstability of prostaglandins causes difficulties in formulating into pharmaceuticals.

CA-A-1 072 413 discloses compositions of 15-50% polyoxyethylene-polyoxypropylene copolymer (Pluronic) which include a therapeutic agent. Int. J. Pharmaceutics 33, 65-69 (1986) discloses crosslinked, polymerized, prostaglandine-containing hydrogels which are prepared from modified Pluronic F-68.

### [Disclosure of the Invention]

In the environment as described, the present inventors have conducted intensive studies, and as a result, have found that stability of prostaglandins in a liquid can be increased by the addition of polyoxyethylene-polyoxypropylene copolymer having a molecular weight of 1000 to 20000 which is among nonionic surfactants, 0.1-10% (w/v), and completed the invention.

The present invention provides liquid compositions containing prostaglandin, 0.1-100 µg/ml, and polyoxyethylene-polyoxypropylene copolymer having a molecular weight of 1000 to 20000, 0.1-10% (w/v).

### (i) Prostaglandin

As the prostaglandin to be used in the present invention, there may be mentioned prostaglandins belonging to groups A, D, E, I, etc.

Specific examples include the following.
- PGA₂: (5Z,13E,15S)-15-hydroxy-9-oxo-prosta-10,13-dienoic acid
- PGD₂: (5Z,13E,15S)-9α,15-dihydroxy-11-oxo-prosta-5,13-dienoic acid
- PGE₁: (13E,15S)-11α,15-dihydroxy-9-oxo-prosta-13-dienoic acid
- PGA₁: (13E,15S)-15-hydroxy-9-oxo-prosta-10,13-dienoic acid
- PGI₂: (5Z,13E,15S)-11α,15-dihydroxy-6,9α-epoxy-prosta-5,13-dienoic acid
- PGE₂: (5Z,13E,15S)-11α,15-dihydroxy-9-oxo-prosta-5,13-dienoic acid
The prostaglandin to be used in the present invention is optionally in the form of salts or esters.

Examples of salts include alkaline earth metal salts (e.g. sodium salt, potassium salt, etc.) and alkali metal salts (e.g. calcium salt, etc.).

Examples of esters include alkyl esters (e.g. alkyl ester having alkyl of 1 to 4 carbon atoms such as methyl ester, ethyl ester, n-propyl ester, n-butyl ester, t-butyl ester, etc.).

Examples of prostaglandin to be used in the present invention include those described in Japanese Patent Unexamined Publication Nos. 216820/1984, 206349/1984 and 13779/1985.

### (ii) Polyoxyethylene-polyoxypropylene copolymer

The polyoxyethylene-polyoxypropylene copolymer to be used in the present invention is not subject to any limitation as far as it has an average molecular weight of 1000 to 20000, preferably 1000 to 5000, with preference given to those comprising ethylene oxide in a proportion of about 10 to 30%, specifically about 20%. Propylene oxide is preferably comprised therein in a proportion of about 90 to 70%, specifically about 80%.

As such compounds, exemplified are those generally referred to by a product name of Pluronic (trade mark) such as the following.
- Pluronic L62: (average molecular weight 2500, ethylene oxide: propylene oxide=20:80, Asahi Denka Kogyo)
- Pluronic L72: (average molecular weight 2850, ethylene oxide: propylene oxide=20:80, Asahi Denka Kogyo)
- Pluronic L122: (average molecular weight 4900, ethylene oxide: propylene oxide=20:80, Asahi Denka Kogyo)

### (iii) Form of pharmaceutical compositions

The form of the liquid compositions of the invention are subject to no limitation as long as prostaglandin mentioned in
(i) and polyoxyethylene-polyoxypropylene copolymer mentioned in
(ii) are comprised therein, and examples thereof include aqueous solutions, liposome solutions and fat emulsion.

The compositions of the invention contain 0.1-100 µg/ml, preferably 1-10 µg/ml of prostaglandin and 0.1-10% (w/v), preferably 1-5% (w/v) of polyoxyethylene-polyoxypropylene copolymer.

The compositions of the invention may further contain known additives.

### (a) Aqueous solutions

As the aqueous solutions, the preferred ones comprise prostaglandin, polyoxyethylene-polyoxypropylene copolymer and a suitable aqueous solvent.

Examples of such an aqueous solvent include injectable distilled water, physiological saline, electrolyte solutions and buffers.

### (b) Liposome solutions

As the liposome solutions, the preferred ones comprise prostaglandin, polyoxyethylene-polyoxypropylene copolymer, a phospholipid, and a suitable aqueous solvent.

Examples of phospholipid include;
① soybean phospholipid or egg yolk phospholipid
② phosphatidylcholine, phosphatidylethanolamine, phosphatidylserine, phosphatidylinositol or sphingomyelin
③ mixture of phospholipids mentioned in ②
   As regards the aqueous solvent, those mentioned in (a) above can be used.

The liposome solution is prepared by a known means [Japanese Patent Unexamined Publication No. 208910/1985, Chemical Abstracts, *93*, 1136f (1980)].

A phospholipid and prostaglandin are dissolved in an organic solvent such as chloroform or ethanol and thoroughly mixed. The vessel is vacuum dried to evaporate the solvent and to allow the phospholipid to adhere thin on the inside wall of the vessel. Thus, a phospholipid membrane is formed. In forming a phospholipid membrane, an antioxidant such as tocopherol (vitamin E) may be added for stabilization of the phospholipid.

The membrane thus formed is added in an appropriate aqueous solvent such as a buffer adjusted to pH 5.5-8, preferably 6-7 (e.g. citrate buffer, phosphate buffer, acetate buffer, physiological saline, etc.) for dissolution, and the mixture is immediately shaken vigorously or stirred for the formation of vesicles by crushing. The vesicles are preferably subjected to an ultrasonic treatment to adjust the diameter to 0.3 µ or less.

In this way, liposomes are formed. The liposomes may be separated and purified by a known means such as centrifugation.

### (c) Lipid emulsion

As the fat emulsion, the preferred ones comprise prostaglandin, polyoxyethylene-polyoxypropylene copolymer, soybean oil, a phospholipid, and a suitable aqueous solvent.

The soybean oil is preferably purified soybean oil with a high purity, and specifically preferred one is purified soybean oil having a high purity (containing 99.9% or more as triglyceride, diglyceride and monoglyceride) which is obtained by further purifying purified soybean oil by the steam distillation method.

As regards the phospholipid and the aqueous solvent, those mentioned in (b) above can be used. The phospholipid is preferably purified egg yolk phospholipid which can be prepared by the conventional fraction method using an organic solvent. That is, crude egg yolk phospholipid is dissolved in cooled n-hexane-acetone, acetone is added thereto little by little with stirring, and the insoluble matters are filtered off, which procedure is conducted in two cycles. Thereafter, the solvent is distilled off to give a purified phospholipid.

A fat emulsion is prepared by a known method (Japanese Patent Unexamined Publication No. 222014/1983).

That is, a given amount of soybean oil, a phospholipid, prostaglandin and other additives mentioned above are mixed and heated to give a solution, which is then homogenized in the conventional homogenizer (e.g. pressure jet homogenizer, ultrasonic homogenizer, etc.) to give a water-in-oil dispersion. A necessary amount of water is added thereto, and homogenization is conducted again as described to give an oil-in-water dispersion. Additives such as a stabilizer, an isotonizing agent, etc. known per se may be added as needed after the formation of a fat emulsion.

The compositions of the invention can be formed into preparations by a known means. That is, heat sterilization, sterilization by filtration, subdivision, charging and so on can be conducted. By employing such method(s), the compositions of the invention can be formulated into injections, and the like.

The compositions of the invention can be administered orally or parenterally (intravenous injection, intra-arterial injection). The dose amount varies depending upon sex, age, body weight, symptoms of patients, etc., but is generally about 1-100 µg daily on a prostaglandin basis in case of intravenous injection.

The compositions of the invention afford increased stability of prostaglandin (specifically stability in a liquid state, stability to heat, stability during storage, etc.) by the addition of polyoxyethylene-polyoxypropylene copolymer, 0.1-10% (w/v).

Thus, the compositions of the invention are considered to be pharmaceutically very useful.

Hereunder follow working examples and experiment examples to illustrate the present invention in detail, which are not to be construed as limitative.

### Example 1

A 5 mM acetate buffer (pH 4.5) containing PGE₁, 5 × 10⁻⁴% (w/v), and polyoxyethylene-polyoxypropylene copolymer (average molecular weight 2850, ethylene oxide:propylene oxide=20:80, product name : Pluronic L-72, Asahi Denka Kogyo), 1.2% (w/v), was prepared.

### Example 2

The same procedure as in Example 1 was repeated except that PGE₂, PGA₁, PGA₂, PGD₂ or PGI₂ was used in place of PGE₁ to give a liquid composition.

### Example 3

PGE₁ (0.25 g), purified egg yolk phospholipid (10 g), polyoxyethylene-polyoxypropylene copolymer (average molecular weight 2850, ethylene oxide:propylene oxide=20:80, product name : Pluronic L-72, Asahi Denka Kogyo) (5 g) and α-tocopherol (10 mg) were dissolved in chloroform (100 ml). The chloroform was evaporated by heating under reduced pressure in a rotary evaporator to form a phospholipid membrane. To this membrane were added 50 mM phosphate buffer (pH 7.0, 100 ml) containing 50 mM sodium chloride, and glass beads, and the mixture was vigorously shaken immediately at room temperature for 20 minutes, which was then subjected to an ultrasonic treatment for 1 hour using a sonicator (Branson Sonic Power, Cell Disrutor #350, output 60 W) with ice-cooling to give a liquid composition. The liquid composition was centrifuged at room temperature (25000 g, 1 hour), and the resulting sediment was collected. The sediment was washed several times with the above-mentioned buffer and sterilized by filtration to give liposomes in a pellet form.

### Example 4

To purified soybean oil (30 g) were added egg yolk lecithin (3.6 g), PGE₁ (900 µg), sodium oleate (0.15 g) and polyoxyethylene-polyoxypropylene copolymer (average molecular weight 2850, ethylene oxide:propylene oxide=20:80, product name : Pluronic L-72, Asahi Denka Kogyo) (3 g), and the mixture was heated for dissolution at 40-75°C. Thereto were added distilled water (200 ml), glycerin (Pharmacopoea Japonica, 7.5 g), and a necessary amount of injectable distilled water (20-40°C) to make the entire amount 300 ml, and the mixture was mixed in a homomixer to give a coarse emulsion.

The obtained coarse emulsion was emulsified by a Manton-Gaulin homogenizer by passing 10 times under pressurization at 120 kg/cm² for the fist step and 500 kg/cm² in total. By these steps, a homogenized fat emulsion containing extremely fine PGE₁ was obtained. The average particle diameter of this emulsion was 0.2-0.4 µ and the emulsion did not contain particles having a diameter of 1 µ or more.

### Experiment Example 1

Effects of various emulsions on PGE₁ stability were examined. A 5 mM acetate buffer (pH 4.5) was used as the control, and when the emulsion was in use, it was used in a proportion of 1.2% (w/v) emulsion/5 mM acetate buffer (pH 4.5).

The treatment conditions were at 90°C and for 4 hours. The PGE₁ content was determined by HPLC. The results are tabled in Table 1.

**Table 1**

| | Emulsion | pH | Residual PGE₁ (%) |
|---|---|---|---|
| before treatment | | 4.50 | 100 |
| after treatment | control | 4.59 | 67.7 |
| | egg yolk lecithin | 4.46 | 28.7 |
| | Pluronic L-62 | 4.56 | 80.4 |
| | Pluronic F-68 | 4.61 | 76.5 |
| | Pluronic F-108 | 4.63 | 76.0 |

### Experiment Example 2

Using the PGE₁-containing emulsion as obtained in Example 3, the relation between the amount added of the emulsion and PGE₁-stabilizing effect was examined. As the emulsion, used was Pluronic L-72 in a proportion of 0-5% (w/v). The treatment conditions were at 90°C and for 4 hours. The PGE₁ content was determined by HPLC. The results are tabled in Table 2.

**Table 2**

| Amount added | Residual PGE₁ |
|---|---|
| 0% (w/v) | 51% |
| 0.1 | 51 |
| 0.2 | 52 |
| 1 | 62 |
| 2 | 70 |
| 5 | 79 |

The PGE₁ content before the treatment was taken as 100%.

While the present invention has been fully described by the foregoing specification including working examples, the present invention can be changed and modified in various manners within the scope of the claims.

## Claims (Claims for the following Contracting State(s): BE, CH, DE, FR, GB, IT, LI, NL, SE)

1. A liquid composition containing prostaglandin, 0.1-100 µg/ml, and polyoxyethylene-polyoxypropylene copolymer having a molecular weight of 1000 to 20000, 0.1-10% (w/v).

2. A liquid composition according to Claim 1, which is in the form of aqueous solutions, liposome solutions or fat emulsions.

3. A liquid composition according to Claim 1, wherein the prostaglandin is at least one member selected from the group consisting of prostaglandins belonging to prostaglandin groups A, D, E and I.

4. A liquid composition according to Claim 1, wherein the prostaglandin is at least one member selected from the group consisting of (5Z,13E,15S)-15-hydroxy-9-oxo-prosta-10,13-dienoic acid, (5Z,13E,15S)-9α,15-dihydroxy-11-oxo-prosta-5,13-dienoic acid, (13E,15S)-11α,15-dihydroxy-9-oxo-prosta-13-dienoic acid, (13E,15S)-15-hydroxy-9-oxo-prosta-10,13-dienoic acid, (5Z,13E,15S)-11α,15-dihydroxy-6,9α-epoxy-prosta-5,13-dienoic acid and (5Z,13E,15S)-11α,15-dihydroxy-9-oxo-prosta-5,13-dienoic acid.

5. A liquid composition according to Claim 1, wherein the polyoxyethylene-polyoxypropylene copolymer has an average molecular weight of 1000 to 5000.

6. A liquid composition according to Claim 1, wherein ethylene oxide is contained in the polyoxyethylene-polyoxypropylene copolymer in a proportion of about 10-30%.

7. A liquid composition according to Claim 1, wherein propylene oxide is contained in the polyoxyethylene-polyoxypropylene copolymer in a proportion of about 90-70%.

8. A liquid composition according to Claim 1, wherein the ethylene oxide is contained in a proportion of about 20% and the propylene oxide is contained in a proportion of about 80% in the polyoxyethylene-polyoxypropylene copolymer.

## Claims (Claims for the following Contracting State(s): ES)

1. A process for preparing a composition containing prostaglandin, 0.1 - 100 µg/ml, and polyoxyethylene-polyoxypropylene copolymer having a molecular weight of 1000 to 20000, 0.1-10 % (w/v), which process comprises admixing appropriate amounts of the prostaglandin with the polyoxyethylene-polyoxypropylene copolymer to reach said concentrations.

2. A process according to claim 1 wherein the prepared liquid composition is in the form of aqueous solutions, liposome solutions or fat emulsion.

3. A process according to Claim 1, wherein the prostaglandin is at least one member selected from the group consisting of prostaglandins belonging to prostaglandin groups A, D, E and I.

4. A process according to Claim 1, wherein the prostaglandin is at least one member selected from the group consisting of (5Z,13E,15S)-15-hydroxy-9-oxo-prosta-10,13-dienoic acid, (5Z,13E,15S)-9α,15-dihydroxy-11-oxo-prosta-5,13-dienoic acid, (13E,15S)-11α,15-dihydroxy-9-oxo-prosta-13-dienoic acid, (13E,15S)-15-hydroxy-9-oxo-prosta-10,13-dienoic acid, (5Z,13E,15S)-11α,15-dihydroxy-6,9α-epoxy-prosta-5,13-dienoic acid and (5Z,13E,15S)-11α,15-dihydroxy-9-oxo-prosta-5,13-dienoic acid.

5. A process according to Claim 1, wherein the polyoxyethylene-polyoxypropylene copolymer has an average molecular weight of 1000 to 5000.

6. A process according to Claim 1, wherein ethylene oxide is contained in the polyoxyethylene-polyoxypropylene copolymer in a proportion of about 10-30%.

7. A process according to Claim 1, wherein propylene oxide is contained in the polyoxyethylene-polyoxypropylene copolymer in a proportion of about 90-70%.

8. A process according to Claim 1, wherein the ethylene oxide is contained in a proportion of about 20% and the propylene oxide is contained in a proportion of about 80% in the polyoxyethylene-polyoxypropylene copolymer.

## Patentansprüche (Patentansprüche für folgende(n) Vertragsstaat(en): BE, CH, DE, FR, GB, IT, LI, NL, SE)

1. Flüssige Zusammensetzung, die 0,1-100 µg/ml Prostaglandin und 0,1-10% (w/v) Polyoxyethylen/Polyoxypropylen-Copolymer mit einem Molekulargewicht von 1000 bis 20 000 enthält.

2. Flüssige Zusammensetzung gemäß Anspruch 1, die in Form von wäßrigen Lösungen, Liposomlösungen oder Fettemulsionen vorliegt.

3. Flüssige Zusammensetzung gemäß Anspruch 1, wobei das Prostaglandin wenigstens ein Element ist, das aus der Gruppe ausgewählt ist, die aus Prostaglandinen besteht, die zu den Prostaglandingruppen A, D, E und I gehören.

4. Flüssige Zusammensetzung gemäß Anspruch 1, wobei das Prostaglandin wenigstens ein Element ist, das aus der Gruppe ausgewählt ist, die aus (5Z,13E,15S)-15-Hydroxy-9-oxoprosta-10,13-diensäure,(5Z,13E,15S)-9α,15-Dihydroxy-11-oxoprosta-5,13-diensäure,(13E,15S)-11α,15-Dihydroxy-9-oxo-prosta-13-diensäure, (13E,15S)-15-Hydroxy-9-oxoprosta-10,13-diensäure, (5Z,13E,15S)-11α,15-Dihydroxy-6,9α-epoxyprosta-5,13-diensäure und (5Z,13E,15S)-11α,15-Dihydroxy-9-oxoprosta-5,13-diensäure besteht.

5. Flüssige Zusammensetzung gemäß Anspruch 1, wobei das Polyoxyethylen/Polyoxypropylen-Copolymer ein mittleres Molekulargewicht von 1000 bis 5000 hat.

6. Flüssige Zusammensetzung gemäß Anspruch 1, wobei Ethylenoxid in dem Polyoxyethylen/Polyoxypropylen-Copolymer in einem Anteil von etwa 10-30% enthalten ist.

7. Flüssige Zusammensetzung gemäß Anspruch 1, wobei Propylenoxid in dem Polyoxyethylen/Polyoxypropylen-Copolymer in einem Anteil von etwa 90-70% enthalten ist.

8. Flüssige Zusammensetzung gemäß Anspruch 1, wobei das Ethylenoxid in einem Anteil von etwa 20% und das Propylenoxid in einem Anteil von etwa 80% in dem Polyoxyethylen/Polyoxypropylen-Copolymer enthalten ist.

## Patentansprüche (Patentansprüche für folgende(n) Vertragsstaat(en): ES)

1. Verfahren zur Herstellung einer Zusammensetzung, die 0,1-100 µg/ml Prostaglandin und 0,1-10% (w/v) Polyoxyethylen/Polyoxypropylen-Copolymer mit einem Molekulargewicht von 1000 bis 20 000 enthält, wobei das Verfahren das Mischen geeigneter Mengen des Prostaglandins und des Polyoxyethylen/Polyoxypropylen-Copolymers umfaßt, so daß man die genannten Konzentrationen erreicht.

2. Verfahren gemäß Anspruch 1, wobei die hergestellte flüssige Zusammensetzung in Form von wäßrigen Lösungen, Liposomlösungen oder Fettemulsionen vorliegt.

3. Verfahren gemäß Anspruch 1, wobei das Prostaglandin wenigstens ein Element ist, das aus der Gruppe ausgewählt ist, die aus Prostaglandinen besteht, die zu den Prostaglandingruppen A, D, E und I gehören.

4. Verfahren gemäß Anspruch 1, wobei das Prostaglandin wenigstens ein Element ist, das aus der Gruppe ausgewählt ist, die aus (5Z,13E,15S)-15-Hydroxy-9-oxoprosta-10,13-diensäure, (5Z,13E,15S)-9α,15-Dihydroxy-11-oxoprosta-5,13-diensäure, (13E,15S)-11α,15-Dihydroxy-9-oxoprosta-13-diensäure, (13E,15S)-15-Hydroxy-9-oxoprosta-10,13-diensäure, (5Z,13E,15S)-11α,15-Dihydroxy-6,9α-epoxyprosta-5,13-diensäure und (5Z,13E,15S)-11α,15-Dihydroxy-9-oxoprosta-5,13-diensäure besteht.

5. Verfahren gemäß Anspruch 1, wobei das Polyoxyethylen/Polyoxypropylen-Copolymer ein mittleres Molekulargewicht von 1000 bis 5000 hat.

6. Verfahren gemäß Anspruch 1, wobei Ethylenoxid in dem Polyoxyethylen/Polyoxypropylen-Copolymer in einem Anteil von etwa 10-30% enthalten ist.

7. Verfahren gemäß Anspruch 1, wobei Propylenoxid in dem Polyoxyethylen/Polyoxypropylen-Copolymer in einem Anteil von etwa 90-70% enthalten ist.

8. Verfahren gemäß Anspruch 1, wobei das Ethylenoxid in einem Anteil von etwa 20% und das Propylenoxid in einem Anteil von etwa 80% in dem Polyoxyethylen/Polyoxypropylen-Copolymer enthalten ist.

## Revendications (Revendications pour l'(les) Etat(s) contractant(s) suivant(s): BE, CH, DE, FR, GB, IT, LI, NL, SE)

1. Composition liquide contenant de la prostaglandine, 0,1-100 µg/ml, et un copolymère de polyoxyéthylène-polyoxypropylène ayant un poids moléculaire de 1000 à 20 000, 0,1-10% (p/v).

2. Composition liquide selon la revendication 1, qui est sous forme de solutions aqueuses, de solutions de liposomes ou d'émulsions grasses.

3. Composition liquide selon la revendication 1, dans laquelle la prostaglandine est au moins un membre choisi dans le groupe constitué par les prostaglandines appartenant aux groupes de prostaglandines A, D, E et I.

4. Composition liquide selon la revendication 1, dans laquelle la prostaglandine est au moins un membre choisi dans le groupe constitué par l'acide (5Z,13E,15S)-15-hydroxy-9-oxo-prosta-10,13-diénoïque, l'acide (5Z,13E,15S)-9α,15-dihydroxy-11-oxo-prosta-5,13-diénoïque, l'acide (13E,15S)-11α,15-dihydroxy-9-oxo-prosta-13-diénoïque, l'acide (13E,15S)-15-hydroxy-9-oxo-prosta-10,13-diénoïque, l'acide (5Z,13E,15S)-11α,15-dihydroxy-6,9α-époxy-prosta-5,13-diénoïque et l'acide (5Z,13E,15S)-11α,15-dihydroxy-9-oxo-prosta-5,13-diénoïque.

5. Composition liquide selon la revendication 1, dans laquelle le copolymère de polyoxyéthylène-polyoxypropylène a un poids moléculaire moyen de 1000 à 5000.

6. Composition liquide selon la revendication 1, dans laquelle l'oxyde d'éthylène est contenu dans le copolymère de polyoxyéthylène-polyoxypropylène dans une proportion d'environ 10-30%.

7. Composition liquide selon la revendication 1, dans laquelle l'oxyde de propylène est contenu dans le copolymère de polyoxyéthylène-polyoxypropylène dans une proportion d'environ 90-70%.

8. Composition liquide selon la revendication 1, dans laquelle l'oxyde d'éthylène est contenu dans une proportion d'environ 20% et l'oxyde de propylène est contenu dans une proportion d'environ 80% dans le copolymère de polyoxyéthylène-polyoxypropylène.

## Revendications (Revendications pour l'(les) Etat(s) contractant(s) suivant(s): ES)

1. Procédé de préparation d'une composition contenant de la prostaglandine, 0,1-100 µg/ml, et un copolymère de polyoxyéthylène-polyoxypropylène ayant un poids moléculaire de 1000 à 20 000, 0,1-10% (p/v), dans lequel on mélange des quantités appropriées de la prostaglandine avec le copolymère de polyoxyéthylène-polyoxypropylène pour atteindre lesdites concentrations.

2. Procédé selon la revendication 1 dans lequel la composition liquide préparée est sous forme de solutions aqueuses, de solutions de liposomes ou d'émulsion grasse.

3. Procédé selon la revendication 1, dans lequel la prostaglandine est au moins un membre choisi dans le groupe constitué par les prostaglandines appartenant aux groupes de prostaglandines A, D, E et I.

4. Procédé selon la revendication 1, dans lequel la prostaglandine est au moins un membre choisi dans le groupe constitué par l'acide (5Z,13E,15S)-15-hydroxy-9-oxo-prosta-10,13-diénoïque, l'acide (5Z,13E,15S)-9α,15-dihydroxy-11-oxo-prosta-5,13-diénoïque, l'acide (13E,15S)-11α,15-dihydroxy-9-oxo-prosta-13-diénoïque, l'acide (13E,15S)-15-hydroxy-9-oxo-prosta-10,13-diénoïque, l'acide (5Z,13E,15S)-11α,15-dihydroxy-6,9α-époxy-prosta-5,13-diénoïque et l'acide (5Z,13E,15S)-11α,15-dihydroxy-9-oxo-prosta-5,13-diénoïque.

5. Procédé selon la revendication 1, dans lequel le copolymère de polyoxyéthylène-polyoxypropylène a un poids moléculaire moyen de 1000 à 5000.

6. Procédé selon la revendication 1, dans lequel l'oxyde d'éthylène est contenu dans le copolymère de polyoxyéthylène-polyoxypropylène dans une proportion d'environ 10-30%.

7. Procédé selon la revendication 1, dans lequel l'oxyde de propylène est contenu dans le copolymère de polyoxyéthylène-polyoxypropylène dans une proportion d'environ 90-70%.

8. Procédé selon la revendication 1, dans lequel l'oxyde d'éthylène est contenu dans une proportion d'environ 20% et l'oxyde de propylène est contenu dans une proportion d'environ 80% dans le copolymère de polyoxyéthylène-polyoxypropylène.
